# EUROPEAN PATENT SPECIFICATION

(11) **EP 1 901 791 B1**
(45) Date of publication and mention of the grant of the patent: **15.08.2012**
(21) Application number: 06779725.8
(22) Date of filing: 16.06.2006
(51) Int. Cl.: A61L 27/52, C08L 5/04, A61L 27/28, A61L 27/22

(54) **BIOCOMPATIBLE COMPOSITION FOR REPLACING/REGENERATING TISSUES**
BIOKOMPATIBLE ZUSAMMENSETZUNG ZUM ERSATZ/ZUR REGENERATION VON GEWEBEN
COMPOSITION BIOCOMPATIBLE POUR REMPLACER/REGENERER DES TISSUS

(30) Priority: 20.06.2005 IT PI20050071
(43) Date of publication of application: 26.03.2008
(73) Proprietor: Calvosa, Giuseppe, 56123 Pisa (IT)
(72) Inventor: BERNARDINI, Nunzia, I-56126 Pisa (IT); LAZZERI, Luigi, I-56040 Crespina (PI) (IT); CASCONE, Maria Grazia, I-55054 Massarosa (LU) (IT)
(74) Representative: Celestino, Marco
(86) International application number: PCT/IB2006/001616
(87) International publication number: WO 2006/136905

(56) References cited:
- WO-A-96/28539
- WO-A-96/40304
- WO-A-02/057355
- WO-A-03/087303
- US-A1- 2001 016 772

## Description

### Field of the invention

The present invention relates to the medical/surgical field and more precisely it relates to a biocompatible composition for replacing/ regenerating biological tissues.

In particular, but not exclusively, said tissues may be both articular tissues, for example tissues of intervertebral discs, or not articular tissues, such as the bones of the skeleton.

In the case of intervertebral discs, the field of interest of the invention is recovering the functionality of the rachis of patients with minimally invasive spine surgery and, in particular, for patients subject to involutional process of the disc owing to degeneration of the nucleus pulposus.

### Background of the invention

In tissue engineering the problem is felt of regenerating tissues that have a biological structure and special biomechanical characteristics, in particular, articular tissues, such as for example tissues of the intervertebral discs, but also not articular tissues, such as the bony tissue of the skeleton.

Articular tissues are present in the rachis and have a relevant function. As known, the rachis consists of a succession of bony structures, the vertebrae, which are linked by fibrous-elastic discs, so-called intervertebral discs. These discs have the function of shock absorbers between the vertebral parts, and consist of fibrocartilaginous tissue organized in order to form: a lamellar peripheral part, normally called annulus, and a soft central part, called nucleus pulposus. The intervertebral discs are firmly connected to the vertebral facing surfaces, called cartilaginous plates, or terminal plates, which carry out a key task within the trophism of discs. The annulus, also-called fibrous ring, consists of a succession of concentric layers of fibrous tissue in which organized fibres of collagen are present. This structure allows the annulus to achieve a high tensile modulus.

In the nucleus pulposus an ovoidal, gelatinous mass is present embedded in the annulus. It has an elastic and resilient consistency and, owing to high water content, is deformable but not compressible. It contains connectival cells and cartilaginous cells, alone or grouped. Also vesiculous cells are present that evidence its derivation from the dorsal cord. The substantial part has a mucoproteic nature; with age the water and mucoproteic content of the core decreases, whereas collagen content increases.

The main component of the amorphous connective tissue matrix of the core is a very hydrofilic polymer called "aggrecane", belonging to the family of proteoglycanes, capable of absorbing a considerable amount of water and then of forming a matrix similar to a gel. The amount of water adsorbed by the core depends not only from the composition of the polymeric matrix but also from the external pressure normally born by the disc. In a healthy disc of a young subject (18 years) the 80% of the core consists of water, but the proportion drops to 69 with age (77 years). The mechanical characteristic of a disc result from the morphology and the soundness of the two structures that make them up: annulus and nucleus pulposus. Such characteristic enable the intervertebral discs to carry out two essential functions: keep the steadiness of the rachis and at the same time give to it enough flexibility.

Degeneration of the disc often starts with structural changes of the amorphous intercellular substance: there is a loss of aggrecane due to proteolysis, the core looses its capacity of linking to water, and then the disc grows thinner loosing its resistance capacity. In turn this causes a higher compression on the ring that makes its structure more susceptible to a delamination and then to a damage. This damage to the annulus speeds up in turn the degeneration of the disc with loss of mechanical resistance of the same.

The repair of an intervertebral disc can be done replacing the whole disc ("total artificial disc") or replacing only the core ("artificial core").

Changing only the core can have many advantages with respect to changing the whole disc. First of all, the original annulus and cartilaginous plates remain and can thus continue to carry out their function. Furthermore, the surgical operation can be much simpler, since a core prosthesis can be implanted with minimally invasive surgery. According to the configuration and to the material chosen, it is possible to implant the device using an endoscopic technique that requires only a small incision of the annulus. All this can reduce the risks associated to the implant with respect to those associated to the implant of a total artificial disc.

However, this type of method can be used only in patients where a degeneration of the disc is at a early or intermediate stage, when the annulus is still perfectly integral. Concerning the material, since metal prostheses would be too hard for the nucleus, many elastomers (both preformed and formed in situ) have been tested. However most of these have problems of biocompatibility and/or inadequate mechanical characteristics.

Examples of the use of these elastomers for replacing the nucleus pulposus, both by means of implants, and by applying a hydrogel, are given in WO 20C4/098756, by means of not natural materials capable of giving problems of biocompatibility.

Cell "scaffolding" systems are also known, as described in US 6872387, for reconstructing tissues in vitro, even if an invasive treatment is required to implant the regenerated tissues in the patients.

WO03/087303 discloses a composition for skin replacement comprising alginate, gelatin and cells like chondrocytes, osteoblasts or fibroblasts wherein the alginate/gelatin is cross linked by CaCl2.

US 2001/016772 discloses a Composite material for cartilage repair which comprises a collagen matrix, alginate and chondrocytes wherein the alginate is cross linked by addition of CaCl2.

WO02/057355 discloses a composition for joints repair which comprises a chemically cross-linkable polysaccharide and a second polysaccharide and chondrocytes wherein the alginate is cross Linked by the addition of CaCl2.

WO 96/403C4 discloses a hydrogel composition for tissue repair like cartilage defects which comprises alginate and cells like chondrocytes and a cross linking agent e.g. calcium, in addition growth factors can be present.

WO 96/28539 discloses a composition for repairing cartilage or bone defects comprising a hydrogel like alginate which is crosslinked with calcium cations and mesenchymal stem cells.

### Summary of the invention

It is then a feature of the present invention to provide a composition for replacing regenerating tissues that is completely biocompatible and can be applied with minimally invasive spine surgery.

It is a particular feature of the invention to provide such a composition that is adapted to replacing regenerating the nucleus pulposus of an intervertebral disc and that allows a full recovery of the functionality of the rachis of an individual affected by pathologies of the rachis through a biocompatible and minimally invasive methodology.

These and other objects are achieved by the biocompatible composition according to the invention for replacing regenerating tissues, particular, articular tissues such as the nucleus pulposus of intervertebral discs, or also other not articular tissues such as bony tissues, this composition comprising:
- a hydrogel matrix comprising a first and a second natural polymer, wherein one among said first and second natural polymers is sodium alginate;
- cells adapted to regenerate said tissues;
- a cross linking agent adapted to cross-link at least one of said natural polymers in situ, thus giving to the biocompatible composition an appropriate resistance;
whose main characteristic is that the cross Linking agent is contained in biodegradable polymeric particles at controlled release, said biodegradable polymeric particles releasing said cross linking agent in a controlled way to electively effects a cross linking action on said sodium alginate.

Preferably, molecules are added to said composition adapted to stimulate the growth and/or the differentiation of cells. In particular, the molecules adapted to stimulate the growth and/or the differentiation cf cells are selected from the group comprised of:
- growth factors;
- molecules of the connective extracellular matrix; insulin;
- ascorbate-2-phosphate.

Advantageously, the composition, as above described, before the cross-linking step has a starting density so that it is injectable.

In particular, the cross linking action on sodium alginate is attained by a cross linking agent contained in biodegradable polymeric particles, released in a controlled way from natural polymers, for example gelatin, or starting from synthetic polymers, for example polylactic acid or copolymers of lactic acid-glycolic acid. Advantageously, the second natural polymer is

Advantageously, the second natural polymer is selected from the group comprised of:
- polymer of nature;
- polymer of proteic nature.

In particular, said polymer of polysaccharidic nature is selected from the group comprised of:
- hyaluronic acid;
- esters of the hyaluronic acid;
- Chitosan.

Preferably, said polymer of proteic nature is selected from the group comprised of:
- collagen;
- gelatin;
- elastin.

In particular, the growth factors are selected from the group comprised of the following families:
- EGF (Epidermal Growth Factor), in particular, TGF-alpha;
- TGF (Transforming Growth Factor), in particular, TGF-beta;
- FGF (Fibroblast Growth Factor);
- PDGF (Platelet-Derived Actor);
- IGF (Insulin-like Growth Factor)

Preferably, the molecules of the connectival extracellular matrix are selected the group comprised of:
- proteoglycanes ;
- laminine;
- fibronectine;
- tenascine.

Preferably, the cells adapted to regenerate the tissue of the core discal are selected from the group comprised of:
- fibroblasts/fibrocytes;
- condroblasts/condrocytes;
- ostecblasts/osteccytes;
- notocordal cells;
- stem mesenchymal cells.

The composition above described has different advantages and, in particular, a lows to minimize the invasivity of the implant, to obtain exactly the desireded shape of the nucleus pulposus and incorporate specific cellular inductor agents. This composition provides, furthermore, the three-dimensional structure on which the cells, suitably selected, carry out the function of restoring in situ the extracellular substances typical of the nucleus pulposus and then of making fibrillar and amorphous substances particularly rich of proteoglycanes .

An example is now given that does not limit the the composition to all the embodiments under the present invention.

### EXAMPLE

To a mixture comprising 5 ml of a 4% solution of sodium alginate in culture medium (D-MEM) (composition 1) and 5 ml of a 0,4% solution of hyaluronic acid in culture medium (D-MEM) (composition 2), under gentle stirring, a 5 ml solution is added of a suspension cf 300 mg of gelatin microparticles (composition 3), prepared according to the described method in culture medium (D-MEM).

Eventually, to the final composition obtained, mixing gently, 5 ml are added of a cellular suspension (dermic fibroblasts) (200.10⁵ cells/ml) in full culture medium charged with FGF.

The procedure above described is carried out at room temperature in conditions of sterility. The process for cross linking the alginate, with subsequent attainment of a three-dimensional hydrogel matrix where are cells vital embedded, starts after about 15 minutes from the addition of composition 3.

Biodegradable microparticles of composition 3 used for attaining a controlled release of the cross linking agent of sodium alginate can be for example prepared as described below.
1. An aqueous phase ("W") is prepared dissolving 0,2 g of gelatin in 20ml of distilled water;
2. An organic phase ("O") is prepared adding 2ml of Span 85 (surfactant of the organic phase) to 110ml of isoctane;
3. An ice bath is prepared where a 250ml balloon is immersed in which the emulsion is made;
4. the aqueous phase (W) is let to drip in the organic phase (O) stirring at 1000 rpm for 10 minutes;
5. 20ml of Tween 85 (surfactant of the aqueous phase) are added while stirring at 1000 rpm continues for further 10 minutes;
6. 5ml of a 25% glutaraldeide aqueous solution and 15 ml of distilled water are let to drip while stirring at 1000 rpm continues for further 15 minutes;
7. Stirring at 500 rpm continues for further 35 minutes;
8. 20ml of 0,1M glycine are added mixing at 500 rpm for further 10 minutes,
9. 4 washings are made with water centrifugating each time at 3000 rpm for 5 minutes;
10. a bath in 10 ml of acetone is made under stirring;
11. a sonication is carried out in a bath at ultrasonic pulses for 60 minutes with periodic stirring of the container that contains the microparticles and the acetone
12. a drying step is effected under a ventilated hood at room temperature obtaining as final product microparticles of gelatin in the form of a fine powder.
13. 234 mg of microparticles thus obtained are added to a 1,4 % calcium chloride aqueous solution. After 4 hours of incubation under gentle stirring, the microparticles are separated by centrifugation and then dried. Their weight after loading is 300 mg.
Notwithstanding in the description and in the examples reference has been made only to replacing/regenerating the nucleus pulposus, it is clear that the invention can be extended also to other articular tissues, but also to not articular tissues such as bony tissues.

## Claims

1. Biocompatible composition for replacing/ regenerating tissues, in particular articular tissues such as the nucleus pulposus of the intervertebral discs, or also not articular tissues such as bony tissues, this composition comprising:
- a hydrogel matrix comprising a first and a second natural Polymer, wherein one among said first and second natural polymers is sodium alginate;
- cells adapted to regenerate said tissues;
- a cross linking agent adapted tc cross-link at least one of said natural polymers in situ, thus giving to the biocompatible composition an appropriate resistance;
**characterised in that**
said cross linking agent is contained in biodegradable polymeric particles at controlled release, said biodegradable polymeric particles releasing said cross linking agent in a controlled way to selectively effects a cross linking action on said sodium alginate.

2. Composition, according to claim 1, wherein molecules are added adapted to stimulate the growth and/or the differentiation of cells, said molecules adapted to stimulate the growth and/or the differentiation cf cells are selected from the group comprised of:
- growth factors;
- molecules of the connectival extracellular matrix;
- insulin;
- ascorbate-2-phosphate.

3. Composition, according to claim 1, **characterized in that** a starting density is chosen before said cross linking agent effects said cross linking action on said sodium alginate such that said composition is injectable.

4. Composition, according to claim. 1, wherein said second natural polymer is selected from the group comprised of:
- a polymer of polysaccharidic nature;
- polymer of proteic nature.

5. Composition, according to claim 4, wherein said polymer of polysaccharidic nature is selected from the group comprised of:
- hyaluronic acid;
- esters of the hyaluronic acid;
- chitosan.

6. Composition, according to claim 4, wherein said polymer of proteic nature is selected from the group comprised of:
- collagen;
- gelatin;
- elastin.

7. Composition, according to claim 2, wherein said growth factors are selected from the group comprised of the following families:
- EGF, in particular, TGF-alpha;
- TGF, in particular, TGF,
- FGF;
- PDGF;
- IGF.

8. Composition, according to claim 2, wherein the molecules of the connectival extracellular matrix are selected from the group comprised of:
- proteoglycanes ;
- laminine;
- fibronectine;
- tenascine.

9. Composition, according to claim 1, wherein said cells adapted to regenerate said tissues are selected from the group comprised of:
- fibroblasts/fibrocytes;
- condroblasts/condrocytes;
- osteoblasts/osteocytes
- notocordal cells;
- stem mesenchymal cells.

## Patentansprüche

1. Biokompatible Zusammensetzung zum Ersetzen/Regenerieren von Geweben, insbesondere Gelenkgeweben wie dem Nucleus pulposus der Bandscheiben, oder auch Nichtgelenkgeweben wie Knochengeweben, wobei diese Zusammensetzung:
eine Hydrogelmatrix, die ein erstes und ein zweites natürliches Polymer umfasst, wobei eines von dem ersten und dem zweiten natürlichen Polymer Natriumalginat ist;
- Zellen, die zur Regeneration der Gewebe angepasst sind
- ein Vernetzungsmittel, das zur Vernetzung von mindestens einem der natürlichen Polymere in situ angepasst ist, wodurch der biokompatiblen Zusammensetzung eine passende Beständigkeit verliehen wird, umfasst;
**dadurch gekennzeichnet, dass**
das Vernetzungsmittel in biologisch abbaubaren Polymerteilchen mit kontrollierter Freisetzung enthalten ist, wobei die biologisch abbaubaren Polymerteilchen das Vernetzungsmittel in kontrollierter Weise derart freisetzen, dass selektiv eine Vernetzungswirkung an dem Natriumalginat bewirkt wird.

2. Zusammensetzung nach Anspruch 1, wobei Moleküle, die zur Stimulierung des Wachstums und/oder zur Differenzierung von Zellen angepasst sind, hinzugefügt sind, wobei die Moleküle, die zur Stimulierung des Wachstums und/oder zur Differenzierung von Zellen angepasst sind, aus der Gruppe von:
- Wachstumsfaktoren,
- Molekülen der extrizellulären Matrix von Bindegewebe,
- Insulin,
- Ascorbat-2-phosphat ausgewählt sind.

3. Zusammensetzung nach Anspruch 1, **dadurch gekennzeichnet, dass**, bevor das Vernetzungsmittel die Vernetzungswirkung an dem Natriumalginat bewirkt, eine Startdichte derart gewählt wird, dass die Zusammensetzung injizierbar ist.

4. Zusammensetzung nach Anspruch 1, wobei das zweite natürliche Polymer aus der Gruppe von:
- einem Polymer der Art eines Polysaccharids,
- einem Polymer der Art eines Proteins ausgewählt ist.

5. Zusammensetzung nach Anspruch 4, wobei das Polymer der Art eines Polysaccharids aus der Gruppe von:
- Hyaluronsäure,
- Estern der Hyaluronsäure,
- Chitosan ausgewählt ist.

6. Zusammensetzung nach Anspruch 4, wobei das Polymer der Art eines Proteins aus der Gruppe von:
- Collagen,
- Gelatine,
- Elastin ausgewählt ist.

7. Zusammensetzung nach Anspruch 2, wobei die Wachstumsfaktoren aus der Gruppe der folgenden Familien:
- EGF, insbesondere TGF-alpha;
- TGF, insbesondere TGF-beta;
- FGF:
- PDGF:
- IGF ausgewählt sind.

8. Zusammensetzung nach Anspruch 2, wobei die Moleküle der extrazellulären Matrix von Bindegewebe aus der Gruppe von:
- Proteoglykanen,
- Laminin,
- Fibronectin,
- Tenascin ausgewählt sind.

9. Zusammensetzung nach Anspruch 1, wobei die Zellen, die zur Regeneration der Gewebe angepasst sind, aus der Gruppe von:
- Fibroblasten/Fibrozyten,
- Chondroblaseen/Chondrozyten,
- Osteoblasten/Osteozyten,
- notochordale Zellen,
- Stamm-Mesenchymzellen ausgewählt sind.

## Revendications

1. Composition biocompatible permettant de remplacer/régénérer des tissus, en particulier des tissus articulaires tels que le noyau pulpeux des disques intervertébraux, ou également des tissus non articulaires, tels des tissus osseux, cette composition comprenant :
- une matrice d'hydrogel comprenant un premier et un second polymère naturel, dans laquelle l'un parmi lesdits premiers et seconds polymères naturels est de l'alginate de sodium ;
- des cellules adaptées pour régénérer lesdits tissus ;
- un agent de réticulation adapté pour réticuler au moins l'un desdits polymères naturels *in situ*, conférant ainsi à la composition biocompatible une résistance appropriée ;
**caractérisée en ce que**
ledit agent de réticulation est contenu dans des particules polymériques biodégradables à libération contrôlée, lesdites particules polymériques biodégradables libérant ledit agent de réticulation de manière contrôlée afin d'effectuer de manière sélective une action de réticulation sur ledit alginate de sodium.

2. Composition selon la revendication 1, dans laquelle des molécules sont ajoutées, adaptées pour stimuler la croissance et/ou la différenciation des cellules, lesdites molécules adaptées pour stimuler la croissance et/ou la différenciation des cellules sont choisies dans le groupe constitué par :
- les facteurs de croissance,
- les molécules de la matrice extracellulaire conjonctive ;
- l'insuline
- l'ascorbate-2-phosphate.

3. Composition selon la revendication 1, **caractérisée en ce qu'**une densité de démarrage est choisie avant que ledit agent de réticulation n'effectue ladite action de réticulation sur ledit alginate de sodium de sorte que ladite composition est injectable.

4. Composition selon la revendication 1, dans laquelle ledit second polymère naturel est choisi dans le groupe constitué par :
- un polymère de nature polysaccharxdique ;
- un polymère de nature protéique.

5. Composition selon la revendication 4, dans laquelle ledit polymère de nature polysaccharidique est choisi dans le groupe constitué par :
- l'acide hyaluronique ;
- les esters de l'acide hyaluronique ;
- le chitosan.

6. Composition selon la revendication 4, dans laquelle ledit polymère de nature protéique est choisi dans le groupe constitué par :
- le collagène ;
- la gélatiné
- l'élastine.

7. Composition selon la revendication 2, dans laquelle lesdits facteurs de croissance sont choisis dans le groupe constitué des familles suivantes :
- EGF, en particulier TGF-alpha ;
- TGF, en particulier TGF-bêta ;
- FGF ;
- PDGF ;
- IGF.

8. Composition selon la revendication 2, dans laquelle les molécules de la matrice extracellulaire conjonctive sont choisies dans le groupe constitué par :
- les protéoglycanes ;
- la laminine ;
- la fibronectine ;
- la ténascine.

9. Composition selon la revendication 1, dans laquelle lesdites cellules adaptées pour régénérer lesdits tissus sont choisies dans le groupe constitué par :
- les fibroblastes/fibrocytes ;
- les chondroblastes/chondrocytes ;
- les ostéoblastes/ostéocytes ;
- les cellules notocordes ;
- les cellules souches mésenchymateuses.
